# DEMANDE DE BREVET EUROPEEN

(11) **EP 0 881 235 A1**
(43) Date de publication de la demande: **02.12.1998**
(21) Numéro de dépôt: 97401142.1
(22) Date de dépôt: 26.05.1997
(51) Int. Cl.: C08F 8/40, C07F 9/535

(54) **Nouveaux ylures de phosphore, leur préparation et leurs utilisations notamment en tant que bases fortes faiblement nucléophiles**

(71) Demandeur: SOCIETE DE CONSEILS DE RECHERCHES ET D'APPLICATIONS SCIENTIFIQUES (S.C.R.A.S.), 75016 Paris (FR); CENTRE NATIONAL DE LA RECHERCHE SCIENTIFIQUE (CNRS), 75794 Paris Cédex 16 (FR)
(72) Inventeur: Bertrand, Guy, 31320 Pechbasque (FR); Bigg, Dennis, 91190 Gif-Sur-Yvette (FR); Cazaux, Jean-Bernard, 30390 Aramon (FR); Goumri, Stéphanie, Appartement A 36, 31400 Toulouse (FR); Guerret, Olivier, 31400 Toulouse (FR)
(74) Mandataire: Bourgouin, André

(57) **Abrégé**

La présente invention concerne de nouveaux ylures de phosphore de formule générale (I) 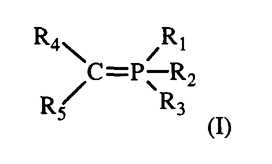 dans laquelle R₁, R₂ et R₃ et R₄ représentent divers groupes variables et R₅ un support polymérique. L'invention concerne également leur préparation et leurs utilisations notamment en tant que bases fortes faiblement nucléophiles.

## Description

La présente invention concerne de nouveaux ylures de phosphore, leur préparation et leurs utilisations notamment en tant que bases fortes faiblement nucléophiles.

Les bases organiques fortes sont généralement nécessaires pour fonctionnaliser des molécules organiques ou organométalliques ; et plus la base est forte, mieux elle piège les protons. Afin de limiter les réactions parasites, il sera préférable et même souhaitable de choisir une base peu nucléophile. Par ailleurs, la facilité de séparer l'acide obtenu du milieu réactionnel est déterminante dans le but de simplifier les étapes de purifications.

Des ylures de phosphore ont été étudiés par Wittig dans les années 50 (Johnson, A. W., 1993, Series, Editor, *Ylides and Imines of phosphorus*, Wiley & Wiley : New York). Ces molécules, assez simples, sont utilisées pour la réaction de Wittig où leur forte nucléophilie est mise en jeu. Mais ces composés n'ont jamais été utilisés en tant que base en synthèse organique, et *a fortiori* en tant que base forte peu nucléophile.

Les bases organiques neutres fortes connues à ce jour sont essentiellement les éponges à protons (Alder, R. et al., *J. Chem. Soc. Chem. Commun.*, 1968, 723), les guanidines, les phosphatranes et les polyphosphazènes.

Les guanidines constituent la classe de bases fortes organiques la plus utilisée en synthèse organique. Les plus connues sont DBN (1,5-diazabicyclo [4.3.0] non-5-ène) et DBU (1,8-diazabicyclo [4.3.0] undec-5-ène). D'autres guanidines sont également mentionnées dans la littérature (Oediger, H. et al., *Synthesis*, 1972, 1972, 593 ; Schwesinger, R., *Angew. Chem. Int. Ed. Engl.*, 1987, 26, 1164). Ces bases trouvent leurs applications dans des réactions d'élimination ou dans des processus d'oligomérisation ou de polymérisation en tant qu'initiateur de réaction (Oediger, H. et al., *Synthesis*, 1972, 1972, 593 ; Wöhrle, D. et al., *Dyes and Pigments*, 1992, 18, 91).

Les phosphatranes sont des composés plus récents (Verkade, J., 1991, *Phosphatranes as proton abstracting reagents*, US patent n°5051533). Leur basicité est très supérieure aux guanidines, ce qui étend leur champ d'application. L'inconvénient de telles phosphines réside dans leur difficulté d'obtention et leur purification.

Quant aux polyphosphazènes, récemment mises au point par Schwesinger, ce sont les molécules organiques les plus basiques montrant une très faible nucléophilie. Elles sont cependant très difficiles à synthétiser et leur coût est très élevé (Schwesinger, R. et al., *Liebigs Ann.*, 1996, 1055 ; Schwesinger, R. et al., *Angew. Chem. Int. Ed. Engl.*, 1991, 30, 1372 ; Schwesinger, R. et al., *Angew. Chem. Int. Ed. Engl.*, 1987, 26, 1167 ; Schwesinger, R. et al., *Angew. Chem. Int. Ed. Engl.*, 1987, 26, 1165 ; Schwesinger, R., *Chimia*, 1985, 39, 269). Leurs applications sont très variées : elles vont de la simple élimination à la benzylation d'oligopeptides (Schwesinger, R., *Chimia*, 1985, 39, 269 ; Pietzonka, T. et al., *Angew. Chem. Int. Ed. Engl.*, 1992, 31, 1481). La basicité de ces molécules est suffisante pour arracher des protons peu acides et leur caractère organique libère des effets de sels gênants dans certaines alkylations (Pietzonka, T. et al., *Chem. Ber.*, 1991, 124, 1837).

Les bases alcalines telles que BuLi, LiHMDS, NaHMDS, LDA représentent également une autre grande famille de bases. Elles sont souvent utilisées avec de bons rendements pour fonctionnaliser des lactames, des cétones ou des bases de Schiff (Stork, G. et al., *J. Org. Chem.*, 1976, 41, 3491 ; Myers, A. et al., *J. Am. Chem. Soc.*, 1994, 116, 9361 ; Myers, A. et al., *J. Am. Chem. Soc.*, 1995, 117, 8488 ; Yaozhong, J. et al., *Synth. Commun.*, 1990, 20, 15 ; Butcher, J., Liverton, N., Selnick, H., Helliot, J., Smith, G., et al., *Tet. Lett.*, 1996, 37, 6685 ; Davis, F., Sheppard, A. et al., *J. Am. Chem. Soc.*, 1990, ). Les effets de sels sont très fréquents et varient souvent d'une réaction à l'autre et ces bases conservent un fort caractère nucléophile même à basse température.(Meyers, A.Kunnen, K., *J. Am. Chem. Soc.*, 1987, 109, 4405).

Le problème est donc de trouver des nouvelles bases fortes peu nucléophiles, faciles à synthétiser et à moindre coût, qui se substitueraient d'une part aux bases communément employées trop nucléophiles et d'autre part aux bases récentes trop onéreuses.

L'invention a ainsi pour objet les produits de formule générale (I) 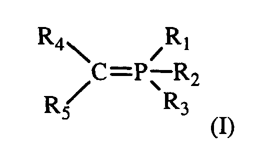
dans laquelle R₁, R₂ et R₃ représentent, indépendamment, un radical alkyle inférieur ; alkoxy inférieur ; aryle éventuellement substitué par un ou plusieurs radicaux choisis parmi les radicaux suivants : alkyle inférieur, haloalkyle, alkoxy inférieur, halogène, cyano, nitro, di(alkyl inférieur) amino ; ou amino de formule R'R''N dans laquelle R' et R'' représentent indépendamment l'un des radicaux non-substitués ou substitués (par un ou plusieurs substituants identiques ou différents) suivants : alkyle inférieur, alkoxy inférieur, cycloalkyle, aryl-alkyle inférieur, aryle, dans lesquels le substituant est un atome d'halogène ou un radical alkyle inférieur, alkoxy inférieur, cyano, nitro ou dialkylamino ;
R₄ représente l'atome d'hydrogène, un radical alkyle inférieur ; et
R₅ représente un support polymérique.

Dans les définitions indiquées ci-dessus, l'expression halogène représente un atome de fluor, de chlore, de brome ou d'iode, de préférence chlore ou brome. L'expression alkyle inférieure représente un radical alkyle ayant de 1 à 6 atomes de carbone linéaire ou ramifié et en particulier un radical alkyle ayant de 1 à 4 atomes de carbone tels que les radicaux méthyle, éthyle, propyle, isopropyle, butyle, isobutyle, sec-butyle et tert-butyle.

Le terme haloalkyle désigne de préférence les radicaux dans lesquels le radical alkyle est tel que défini ci-dessus et est substitué par un ou plusieurs atomes d'halogène tel que défini ci-dessus comme, par exemple, bromoéthyle, trifluorométhyle, trifluoroéthyle ou encore pentafluoroéthyle. Les radicaux alkoxy inférieurs peuvent correspondre aux radicaux alkyle indiqués ci-dessus. Parmi les radicaux linéaires, on peut citer les radicaux méthoxy, éthoxy, propyloxy, n-butyloxy ou n-hexyloxy. Parmi les radicaux alkoxy ramifiés, on peut citer les radicaux isopropyloxy, sec-butyloxy, isobutyloxy, ter-butyloxy, isopentyloxy, neopentyloxy ou ter-pentyloxy.

Le terme cycloalkyle inclut tout fragment hydrocarboné cyclique non-aromatique ayant de 3 à 10 atomes de carbones et de préférence cyclopropyle, cyclobutyle, cyclopentyle ou cyclohexyle.

Le terme aryle désigne les radicaux insaturés, monocycliques ou constitués de cycles condensés, carbocycliques ou hétérocycliques, étant entendu que les radicaux hétérocycliques peuvent inclure un ou plusieurs hétéroatomes identiques ou différents choisis parmi l'oxygène, le soufre ou l'azote. Des exemples de tels groupes incluent les radicaux phényle, thiényle, furyle, pyridyle, pyrimidyle, pyrrolyle, thiazolyle, isothiazolyle, diazolyle, triazolyle, thiatriazolyle, oxazolyle, benzothiényle, benzofuryle, benzopyrtolyle, benzimidazolyle, benzaxozolyle, indolyle, purinyle, naphtyle, thionaphtyle, phénanthrényle, anthracényle, biphényle, indényle, quinolyle, isoquinolyle ou quinolizinyle.

L'expression faiblement nucléophile signifie non nucléophile envers d'autres centres que des protons. Le terme base forte correspond au terme communément utilisé par l'homme de l'art dans le domaine technique considéré. Le support polymérique peut être, par exemple, de type méthacrylique, acrylique ou styrénique.

L'invention a plus particulièrement pour objet les produits de formule générale I telle que définie ci-dessus caractérisée en ce que R₁, R₂ et R₃ représentent, indépendamment, un radical alkoxy inférieur ou amino de formule RR'N telle que définie ci-dessus ; et R₄ représente l'atome d'hydrogène. De préférence, R₁, R₂ et R₃ représentent, indépendamment, le radical méthoxy, éthoxy, diméthylamino, (méthyl)(éthyl)amino, diéthylamino.

L'invention a plus particulièrement pour objet les produits de formule générale I telle que définie ci-dessus caractérisé en ce que le support polymérique R₅ est de type méthacrylique, acrylique tel que le polymère expansine®, ou polystyrénique. De préférence, le support polymérique de type polystyrénique est de formule générale (s) 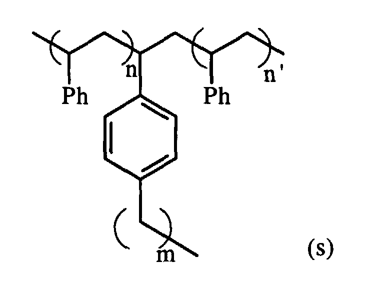 dans laquelle n, n', m sont des entiers supérieurs ou égaux à un. Le support polymérique de formule (s) peut provenir du polymère correspondant de formule (p) 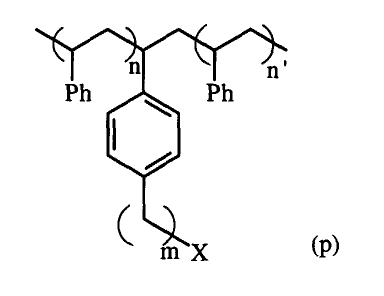 dans laquelle n, n', m sont des entiers supérieurs ou égaux à un et X représente un groupe partant. En tant que groupe partant, X peut être, par exemple, un atome d'halogène, le radical oxycarbonyle, oxysulfonyle ou oxyboronyle. De préférence, le support polymérique R₅ provient d'un polymère polystyrénique de formule générale (p) telle que définie ci-dessus dans laquelle X représente l'atome de chlore et m est égal à 1, et plus particulièrement de la résine de Merrifield.

L'invention a également pour objet un procédé de préparation des produits de formule générale I telle que définie ci-dessus, caractérisé en ce que
l'on fait réagir une phosphine de formule générale (1)

PR₁R₂R₃ (1)

dans laquelle R₁, R₂ et R₃ ont la signification indiquée ci-dessus, avec un composé de formule générale (2) 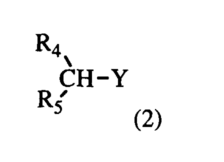 dans laquelle R₄ et R₅ ont la signification indiquée ci-dessus et Y est tel que Y⁻ représente tout anion, pour obtenir le produit de formule générale (3) 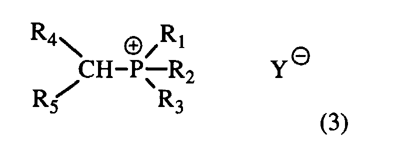 produit (3) ainsi obtenu que l'on traite avec une base forte pour obtenir un produit de formule I.

La préparation du composé (3), à partir des composés (1) et (2) s'effectue de préférence sous atmosphère inerte, comme par exemple sous atmosphère d'argon, dans un solvant polaire tel que 1,2-dichloroéthane, l'acétonitrile, le diméthoxyéthane ou le diéthoxyméthane. Le composé (2) est en général en excès stoechiométrique par rapport au composé (1). Dans ce composé (2), Y est tel que Y⁻ représente tout anion connu de l'homme de l'art tel que, par exemple, halogénures, triflate. Le milieu réactionnel est alors agité au reflux du solvant. Après les étapes classiques de lavage, le composé (3) est séché sous vide. Afin d'obtenir le produit (I), le produit (3) ainsi obtenu est traité, à température ambiante, en présence d'une base forte. La base forte peut être par exemple une base lithiée telle les lithiens d'alkyle, et plus particulièrement le butyllithium, un hydrure de sodium ou de potassium, un amidure ou un alcoolate. Le solvant peut être choisi parmi le pentane, l'éther, le tétrahydrofurane, le diméthoxyéthane ou le diéthoxyméthane, solvant dans lequel la base forte est stable. Le mélange obtenu est agité pendant quelques heures. Le polymère (I) est lavé sous vide puis séché. Il peut ensuite être utilisé directement sans autre purification.

Le composé de formule (I) dans laquelle R₄ ne représente pas l'atome d'hydrogène, peut être obtenu en faisant réagir le composé (I) correspondant dans lequel R₄ représente l'atome d'hydrogène, avec un composé de formule R₄X' dans laquelle X' représente un atome d'halogène et R₄ est tel que défini ci-dessus mais ne représente pas l'atome d'hydrogène, à une température comprise entre -10 et +10° C, de préférence à 0° C, dans un solvant tel que le THF. Après filtration et lavage le produit ainsi obtenu peut être traité avec une base forte pour obtenir le composé (I) souhaité.

L'invention concerne également l'utilisation de produits de formule générale I' 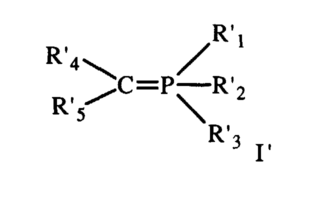 dans laquelle
R'₁, R'₂ et R'₃ représentent, indépendamment, un radical alkyle inférieur ; alkoxy inférieur ; aryle éventuellement substitué par un ou plusieurs radicaux choisis parmi les radicaux suivants : alkyle inférieur, haloalkyle, alkoxy inférieur, halogène, cyano, nitro ou di(alkyl inférieur) amino ; ou amino de formule R'R''N dans laquelle R' et R'' représentent indépendamment l'un des radicaux non-substitués ou substitués (par un ou plusieurs substituants identiques ou différents) suivants : alkyle inférieur, alkoxy inférieur, cycloalkyle, aryl-alkyle inférieur, aryle, dans lesquels le substituant est un atome d'halogène ou un radical alkyle inférieur, alkoxy inférieur, cyano, nitro ou dialkylamino ;
R'₄ représente l'atome d'hydrogène, un radical alkyle inférieur ou alkoxy inférieur ;
R'₅ représente l'atome d'hydrogène, un radical alkyle inférieur, alkoxy inférieur ou un support polymérique ;
en tant que base forte faiblement nucléophile.

L'invention concerne plus particulièrement l'utilisation, en tant que base forte faiblement nucléophile, de produits de formule générale l'telle que définie ci-dessus caractérisée en ce que R'₁, R'₂ et R'₃ représentent, indépendamment, un radical alkoxy inférieur ou amino de formule RR'N telle que définie ci-dessus ; et R'₄ représente l'atome d'hydrogène. De préférence, R'₁, R'₂ et R'₃ représentent, indépendamment, le radical méthoxy, éthoxy, diméthylamino, (méthyl)(éthyl)amino, diéthylamino.

L'invention concerne plus particulièrement également l'utilisation, en tant que base forte faiblement nucléophile, de produits de formule générale I' telle que définie ci-dessus caractérisée en ce que le support polymérique R'₅ est de type méthacrylique, acrylique tel que le polymère expansine®, ou polystyrénique. De préférence, le support polymérique de type polystyrénique est de formule générale (s) telle que définie ci-dessus.

L'invention a plus particulièrement pour objet l'utilisation des composés de formule I' telle que définie ci-dessus, en tant que base forte faiblement nucléophile dans les réactions de N-alkylation telles que, par exemple, les réactions de N-alkylation des lactames, des succinimides, d'oligopeptides et de benzodiazépines.

L'invention a plus particulièrement pour objet également l'utilisation des produits de formule générale I' telle que définie ci-dessus en tant que base forte faiblement nucléophile dans les réactions de C-alkylation telles que, par exemple, les réactions de C-alkylation des lactames, des succinimides, de bases de Schiff et de benzodiazépines.

L'invention a plus particulièrement pour objet l'utilisation des produits répondant aux formules suivantes :
- copolymère styrène/divinylbenzène-tris(diméthylamino)méthylènephosphorane;
- tris(diméthylamino)-C-diméthylméthylène phosphorane.
en tant que base forte faiblement nucléophile, et notamment dans les réactions de N-alkylation ou de C-alkylation.

L'invention a également pour objet, à titre de produits industriels nouveaux, et notamment à titre de produits industriels nouveaux destinés à la préparation des produits de formule (I), les produits de formule (3) telles que décrites ci-dessus.

Les composés de formule (1) et (2) sont généralement des produits commerciaux et peuvent être obtenus selon les méthodes classiques connues de l'homme de l'art. Les composés de formule (I') dans laquelle R'₅ représente l'atome d'hydrogène, un radical alkyle inférieur ou alkoxy inférieur, peuvent être préparés selon les méthodes classiques connues de l'homme de l'art (Johnson, A. W., 1993, Series, Editor, *Ylides and Imines of phosphorus*, Wiley & Wiley : New York).

Les exemples suivants sont présentés pour illustrer les procédures ci-dessus et ne doivent en aucun cas être considérés comme une limite à la portée de l'invention.

### PARTIE EXPÉRIMENTALE :

### Exemple 1 : Préparation d'un ylure supporté

### Etape 1a : Préparation du composé (3)

7 g de résine de Merrifield (1 meq/g) sont mis sous atmosphère inerte d'argon. Une solution de 21 mmol de P(NMe₂)₃ dans 100 ml de dichloroéthane est ajoutée et maintenue sous agitation magnétique pendant 5 jours à température ambiante. Le polymère est ensuite lavé plusieurs fois avec du dichlorométhane pour entraîner l'excès de phosphine. Il est ensuite séché sous vide jusqu'à ce que la masse du polymère obtenu ne change plus (8 g). Le composé (3) est obtenu sous forme d'un fin sable blanc. (Pf (dec.) : 325° C).

Le taux de greffage est contrôlé par la réactivité du polymère obtenu. Le solvant utilisé pour le greffage peut aussi être du diméthoxyéthane.

### 1b : préparation du composé (I)

A 1 g du composé (3) en suspension dans 20 ml de tétrahydrofurane (THF) sont ajoutés 1,1 mmol de butyllithium à température ambiante. Le mélange devient rouge et on observe on dégagement de chaleur. L'agitation est maintenue pendant une heure. On rajoute alors 40 ml de THF et on récupère le polymère par filtration. On lave ensuite le polymère avec 40 ml de THF. L'opération est répétée deux fois. On obtient alors une poudre orangée qui est directement utilisée dans les réactions de déprotonation. Après chaque utilisation le polymère précurseur (3) est récupéré par filtration et peut être de nouveau transformé en ylure supporté.

### Exemple 2 : Utilisation des composés selon l'invention dans une réaction de N-alkylation.

### Exemple 2a : N-fonctionnalisation du norvalium

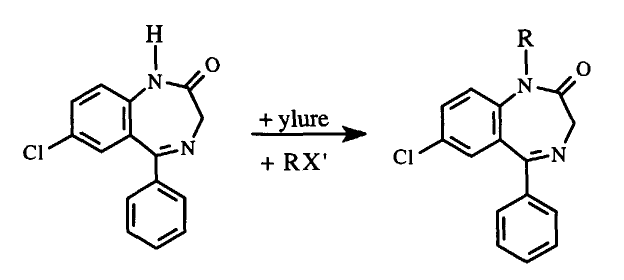

1 g de l'ylure supporté tel que préparé selon l'exemple 1, est agité dans 20 ml de THF. On ajoute une solution de 0,8 mmole de norvalium dans 10 ml de THF et le mélange est maintenu sous agitation pendant une demi-heure. On ajoute alors 1 mmol d'électrophile distillé de formule RX' dans laquelle X' représente un atome d'halogène et R un radical alkyle éventuellement substitué ou SiMe₃, et on laisse le mélange sous agitation pendant 4 heures. La solution est alors séparée des polymères, les solvants sont évaporés sous vide et l'huile obtenue est reprise dans les solvants indiqués ci-dessous. Le polymère est quant à lui lavé et peut être réutilisé ultérieurement.

| Ex | RX' | Solvant | Rendement (%) |
|---|---|---|---|
| 2a₁ | Me₃SiCl | Pentane | 80 |
| 2a₂ | BrCH₂CO₂tBu | Et₂O | 70 |
| 2a₃ | PhCH₂Br | Et₂O | 76 |

### Exemple 2a₁:

RMN ¹H (CDCl₃) : 0,30 (s, 9H, CH₃) ; 4,10 (s large, 2H, CH₂) ; 7,30 (m, 8H, Hₐᵣₒₘ).
RMN ¹³C (CDCl₃) : -0,15 (s, CH₃) ; 53,71 (s, CH₂) ; 127,62-128,11 (s, Cq) ; 129,63-130,52 (s, CHₐᵣₒₘ) ; 169,50 ; 169,52 (s, CO et CN).

### Exemple 2a₂:

RMN ¹H (CDCl₃) : 1,40 (s, 9H, CH₃) ; 3,80 (d, ²J_{(HH)} = 10,7 Hz, 1H, CH₂) ; 4,21 (d, ²J_{(HH)} = 15,0 Hz, 1H, CH₂COOtBu) ; 4,45 (d, ²J_{(HH)} = 15,0 Hz, 1H, CH₂COOtBu); 4,85 (d, ²J_{(HH)} = 10,7 Hz, 1H, CH₂) ; 7,40 (m, 8H, Hₐᵣₒₘ).
RMN ¹³C (CDCl₃) : 27,65 (s, CH₃) ; 51,03 (s, CH₂-COOtBu) ; 51,03 (s, CH₂-COOtBu) ; 55,88 (s, CH₂) ; 83,50 (s, Cq(CH₃)₃) ; 123,63-131,98 (s, CHₐᵣₒₘ) ; 130,23 ; 130,64 ; 137,85 ; 140,56 (s, Cᵢₚₛₒ) ; 168,30 ; 170,26 ; 170,58 (s, CO, COO et CN).

### Exemple 2a₃:

RMN ¹H (CDCl₃) : 1,41 (s, 9H, CH₃) ; 3,80 (d, ²J_{(HH)} = 10,5 Hz, 1H, CH₂) ; 4,23 (d, ²J_{(HH)} = 17,2 Hz, 1H, CH₂COOtBu) ; 4,51 (d, ²J_{(HH)} = 17,2 Hz, 1H, CH₂COOtBu) ; 4,96 (d, ²J_{(HH)} = 10,5 Hz, 1H, CH₂) ; 7,21-7,60 (m, 8H, Hₐᵣₒₘ).
RMN ¹³C (CDCl₃) : 17,16 (s, CH₃) ; 27,81 (s, CH-CH₃) ; 50,65 (s, CH₂) ; 58,46 (s, CH) ; 82,25 (s, Cq(CH₃)₃) ; 122,29-131,37 (s, CHₐᵣₒₘ) ; 129,41 ; 130,82 ; 138,04 ; 141,18 (s, Cq) ; 167,04 ; 167,59 ; 170,50 (s, CO, CN et COO).

### Exemple 2b : N-alkylation de lactames

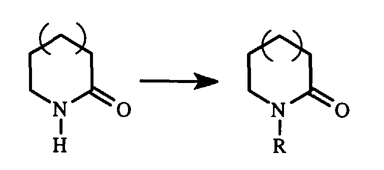

A une solution de 3 mmoles de substrat dans 14 ml de THF, on ajoute lentement, à 25° C, une solution de 3,6 mmoles de (Me₂N)₃P=C(CH₃)₂ dans 17 ml de THF. Le mélange réactionnel est agité à 25°C pendant une heure puis, 3,6 mmoles d'électrophile distillé de formule RX' dans laquelle X' représente un atome d'halogène et R un radical alkyle éventuellement substitué, sont additionnées. L'agitation est maintenue 3 heures supplémentaires. La solution surnageante est séparée des sels de phosphonium formés par extraction avec 60 ml d'éther. Après concentration, l'huile obtenue est purifiée sur colonne chromatographique.

| RX' | Eluant | Rendement (%) |
|---|---|---|
| CH₃I | Et₂O/MeOH 85/15 | 60 |
| BrCH₂CO₂tBu | Et₂O | 70 |
| PhCH₂Br | Et₂O/MeOH 30/70 | 71 |

### Caractérisation de la lactame N-alkylée :

- R = CH₃-: RMN ¹H (CDCl₃) : 1,82 (m, 4H, CH₂-CH₂) ; 2,47 (m, 2H, CH₂-CO) ; 2,98 (s, 3H, CH₃-N) ; 3,31 (m, 2H, CH₂-N).
I.R. (CDCl₃) : 1642 cm⁻¹ (CO).
- R = tBuOC(O)CH₂-: RMN ¹H (CDCl₃) : 1,43 (s, 9H, CH₃) ; 1,80 (m, 4H, CH₂-CH₂) ; 2,55 (m, 2H, CH₂-CO) ; 3,35 (m, 2H, CH₂-N) ; 4,09 (s, 2H, CH₂-COO).
RMN ¹³C (CDCl₃) : 20,44 ; 22,42 (s, CH₂) ; 27,73 (s, CH₃) ; 31,72 (s, CH₂-CO) ; 49,21 ; 49,90 (s, CH₂-N) ; 82,49 (s, C(CH₃)₃) ; 168,59 ; 172,37 (s, CO).
- R = PhCH₂-: RMN ¹H (CDCl₃) : 1,70 (m, 4H, CH₂-CH₂) ; 2,45 (m, 2H, CH₂-CO) ; 3,14 (m, 2H, CH₂-N) ; 4,53 (s, 2H, CH₂Ph) ; 7,20 (m, 5H, Hₐᵣₒₘ).
RMN ¹³C (CDCl₃) : 20,70 ; 22,60 (s, CH₂) ; 31,89 (s, CH₂-CO) ; 47,08 ; 49,91 (s, CH₂-N) ; 127,15 ; 127,57 ; 128,37 (s, CHₐᵣₒₘ) ; 136,56 (s, Cᵢₚₛₒ) ; 170,77 (s, CO)

### Exemple 2c : N-benzylation de peptides

La N-t-Boc-L-Leucine et le chlorhydrate de l'ester méthylique de la L-phénylalanine, sont préalablement couplés en présence d'un agent de couplage BrOP afin d'obtenir le dipeptide correspondant.

A une solution d'un équivalent du dipeptide ainsi obtenu (1,60 mmoles) dans 9 ml de THF, on ajoute lentement, à 25°C, 4 équivalents d'ylure (5,85 mmoles) dans 33 ml de THF. Le mélange réactionnel est agité à 25°C pendant deux heures puis, 4 équivalents de bromure de benzyle (5,85 mmoles) sont additionnés. L'agitation est maintenue 3 heures supplémentaires. La solution surnageante est séparée des sels de phosphonium formés par extraction avec 60 ml d'éther. Après concentration, l'huile obtenue fait l'objet d'une purification sur colonne de silice (éluant : éther/hexane 50/50).

Le produit de monobenzylation est obtenu avec un rendement de 12% et celui de dibenzylation avec un rendement de 28%. Ces composés ont fait l'objet d' analyses par chromatographie en phase gazeuse couplé spectrométrie de masse impact électronique.

### Exemple 3 : Utilisation des composés selon l'invention dans une réaction de C-alkylation.

### Exemple 3a : C-benzylation de la N-méthyl-2-pyrrolidinone

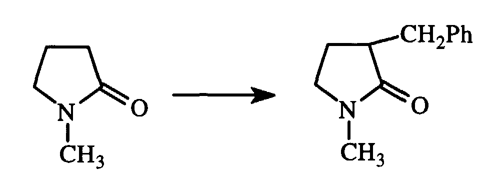

A une solution de 5 mmoles de N-méthyl-2-pyrrolidinone dans 23 ml de THF, on ajoute 6 mmoles de (Me₂N)₃P=C(CH₃)₂ dans 27 ml de THF. La solution est chauffée à 45° C pendant 6 heures puis, 6 mmoles de bromure de benzyle sont additionnées et l'agitation est maintenue une heure supplémentaire à 45°C. On laisse le mélange réactionnel revenir lentement à température ambiante. Après extraction avec 60 ml d'éther, filtration et évaporation du solvant, l'huile obtenue est purifiée sur colonne chromatographique (éluant : éther/méthanol 95/5).
RMN ¹H (CDCl₃) : 2,00 (m, 2H, CH₂) ; 2,63 (m, 2H, CH₂-N) ; 2,79 (s, 3H, CH₃-N) ; 3,10 (m, 3H, CH et CH₂Ph) ; 7,19 (m, 5H, Hₐᵣₒₘ).
RMN ¹³C (CDCl₃) : 23,88 (s, CH₂) ; 29,59 (s, CH₃-N) ; 36,99 (s, CH₂) ; 43,27 (s, CH) ; 47,39 (s, CH₂-N) ; 126,17, 128,28, 128,88 (s, CHₐᵣₒₘ) ; 139,29 (s, Cᵢₚₛₒ) ; 175,72 (s, CO).

### Exemple 3b : C-alkylation en position 3 de benzodiazépines selon le schéma suivant

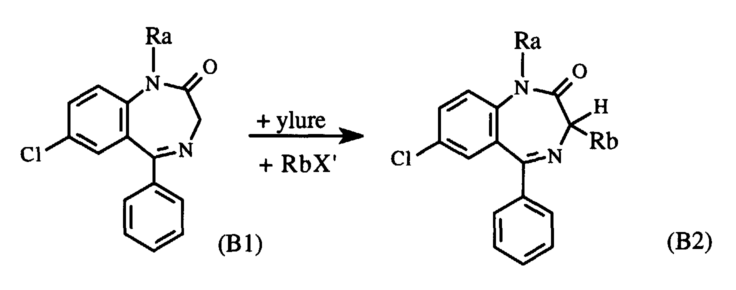

A une solution de 1 mmole du composé (B1) dans 5 ml de THF, on ajoute, à -78° C, 1,5 mmoles d'ylure (Me₂N)₃P=C(CH₃)₂ dans 7,5 ml de THF. Le mélange réactionnel est agité pendant 15 minutes puis, 1,5 mmoles du composé de formule RbX' dans laquelle X' représente un atome d'halogène et Rb un radical alkyl éventuellement substitué, sont additionnées. L'agitation est maintenue 30 minutes supplémentaires. Après extraction avec 60 ml d'éther, filtration et évaporation du solvant, l'huile est purifiée sur colonne chromatographique (éluant : pentane/éther 50/50). Le composé de formule (B2) est obtenu sous forme d'une poudre.

Les résultats obtenus selon la valeur de Ra et Rb sont résumés dans le tableau ci-dessous.

| Ex | Ra | Rb | Rendement (%) |
|---|---|---|---|
| 3b₁ | -CH₂Ph | -CH₂Ph | 38 |
| 3b₂ | -CH₂Ph | -CH₂CO₂tBu | 42 |
| 3b₃ | -CH₂Ph | -CH₃ | 67 |
| 3b₄ | -CH₃ | -CH₂Ph | 55 |
| 3b₅ | -CH₃ | -CH₂CO₂tBu | 42 |
| 3b₆ | -CH₃ | -CH₃ | 43 |
| 3b₇ | -CH₂CO₂tBu | -CH₂CO₂tBu | 48 |
| 3b₈ | -CH₂CO₂tBu | -CH₂Ph | 39 |
| 3b₉ | -CH₂CO₂tBu | -CH₃ | 54 |

### Caractéristiques des composés :

### Exemple 3b₁: point de fusion 87° C

RMN ¹H (CDCl₃) : 3,65 (m, 2H, CH₂Ph) ; 3,87 (m, 1H, CH) ; 4,68 (d, ²J_{(HH)}=15,3 Hz, 1H, N-CH₂Ph) ; 5,68 (d, ²J_{(HH)} = 15,3 Hz, 1H, N-CH₂Ph) ; 7,20 (m, 18H, Hₐᵣₒₘ).
RMN ¹³C (CDCl₃) : 37,95 (s, CH-CH₂Ph) ; 49,95 (s, N-CH₂Ph) ; 65,27 (s, CH-CH₂Ph) ; 123,92-131,24 (s, CHₐᵣₒₘ) ; 129,43, 131,99, 136,27, 137,94, 138,99, 140,05 (s, Cq) ; 167,43 ; 169,05 (s, CO et CN).

### Exemple 3b₂: point de fusion 166° C

RMN ¹H (CDCl₃) : 1,47 (s, 9H, CH₃) ; 3,25 (dd, ²J_{(HH)} = 16,9 Hz, ²J_{(HH)} = 7,5 Hz, H_{B}, CH₂) ; 3,43 (dd, ²J_{(HH)} = 16,9 Hz, ²J_{(HH)} = 6,5 Hz, H_{B',} CH₂) ; 4,19 (dd, ³J_{(HH)} = 7,6 Hz, ³J_{(HH)} = 6,5 Hz, H_{A,} CH) ; 4,75 (d, ²J_{(HH)} = 15,5 Hz, H_{A}, N-CH₂Ph) ; 5,60 (d, ²J_{(HH)} = 15,5 Hz, H_{B}, N-CH₂Ph) ; 6,97-7,43 (m, 13H, Hₐᵣₒₘ).
RMN ¹³C (CDCl₃) :28,14 (s, CH₃) ; 37,80 (s, CH₂COOtBu) ; 50,28 (s, N-CH₂Ph) ; 60,62 (s, CH); 80,70 (s, Cq(CH₃)₃) ; 124,01-131,38 (s, CHₐᵣₒₘ) ; 129,54 ; 132,11 ; 136,20 ; 137,90 ; 140,31 (s, Cq) ; 167,50 ; 169,02 ; 171,30 (s, CO, CN et COO).

### Exemple 3b₃: point de fusion 164° C

RMN ¹H (CDCl₃) : 1,77 (d, ³J_{(HH)} = 6,4 Hz, 3H, CH₃) ; 3,81 (q, ³J_{(HH)} = 6,4 Hz, 1H, CH) ; 4,68 (d, ²J_{(HH)} = 15,5 Hz, H_{A}, CH₂Ph) ; 5,70 (d, ²J_{(HH)} = 15,5 Hz, H_{B}, CH₂Ph) ; 6,99-7,42 (m, 13H, Hₐᵣₒₘ).
RMN ¹³C (CDCl₃) : 17,32 (s, CH₃) ; 49,82 (s, CH₂Ph) ; 58,81 (s, CH) ; 123,94-131,30 (s, CHₐᵣₒₘ) ; 130,38 ; 131,07 ; 136,39 ; 137,93 ; 140,20 (s, Cq) ; 167,16 ; 170,35 (s, CO et CN).

### Exemple 3b₄: point de fusion 85° C

RMN ¹H (CDCl₃) : 3,40 (s, 3H, N-CH₃) ; 3,56-3,77 (m, 3H, CH et CH₂Ph) ; 7,19-7,55 (m, 13H, Hₐᵣₒₘ).
RMN ¹³C (CDCl₃) : 35,28 (s, N-CH₃) ; 38,10 (s, CH₂Ph) ; 65,27 (s, CH) ; 122,77-131,45 (s, CHₐᵣₒₘ) ; 129,16 ; 130,32 ; 138,15 ; 139,21 ; 142,15 (s, Cq) ; 168,01 ; 170,05 (s, CO et CN).

### Exemple 3b₅: point de fusion 141° C

RMN ¹H (CDCl₃) : 1,45 (s, 9H, CH₃) ; 3,40 (s, 3H, N-CH₃) ; 3,11 (dd, ²J_{(HH)} = 17,1 Hz, ²J_{(HH)} = 7,0 Hz, H_{B,} CH₂) ; 3,39 (dd, ²J_{(HH)} = 17,1 Hz, ²J_{(HH)} = 6,8 Hz, H_{B'}, CH₂); 4,07 (dd, ³J_{(HH)} = 7,0 Hz, ³J_{(HH)} = 6,8 Hz, H_{A,} CH) ; 7,25-7,57 (m, 8H, Hₐᵣₒₘ).
RMN ¹³C (CDCl₃) : 28,12 (s, CH₃) ; 35,21 (s, N-CH₃) ; 37,91 (s, CH₂) ; 60,56 (s, CH) ; 80,59 (s, C_{q}(CH₃)₃) ; 122,83-130,36 (s, CHₐᵣₒₘ) ; 131,52 ; 137,97 ; 139,40 ; 142,20 (s, C_{q}) ; 167,20 ; 169,70 ; 171,25 (s, CO, CN et COO).

### Exemple 3b₆: point de fusion 76° C

RMN ¹H (CDCl₃) : 1,71 (d, ³J_{(HH)} = 6,5 Hz, 3H, CH₃) ; 3,39 (s, 3H, N-CH₃) ; 3,70 (q, ³J_{(HH)} = 6,5 Hz, CH) ; 7,25-7,60 (m, 8H, Hₐᵣₒₘ).
RMN ¹³C (CDCl₃) : 17,43 (s, CH₃) ; 35,15 (s, N-CH₃) ; 58,90 (s, CH) ; 122,59-130,54 (s, CHₐᵣₒₘ) ; 129,11 ; 129,63 ; 131,38 ; 138,13 (s, Cq) ; 166,85 ; 171,21 (s, CO et CN).

### Exemple 3b₇: point de fusion 128° C

RMN ¹H (CDCl₃) : 1,40 (s, 9H, CH₃) ; 1,44 (s, 9H, CH₃) ; 3,14 (dd, ²J_{(HH)} = 16,9 Hz, ²J_{(HH)} = 6,8Hz, 1H, H_{B}) ; 3,43 (dd, ²J_{(HH)} = 16,9 Hz, ²J_{(HH)} = 7,0 Hz, 1H, H_{B'}) ; 4,15 (dd, ³J_{(HH)} = 7,0 Hz, ³J_{(HH)} = 6,8 Hz, 1H, H_{A}) ; 4,20 (d, ²J_{(HH)} = 17,1 Hz, 1H, CH₂COOtBu) ; 4,48 (d, ²J_{(HH)} = 17,1 Hz, 1H, CH₂COOtBu) ; 7,25-7,60 (m, 8H, Hₐᵣₒₘ).
RMN ¹³C (CDCl₃) : 27,90 ; 28,06 (s, CH₃) ; 7,82 (s, CH₂) ; 50,81 (s, N-CH₂) ; 60,33 (s, CH) ; 80,61 ; 82,43 (s, Cq(CH₃)₃) ; 123,02-131,64 (s, CHₐᵣₒₘ) ; 129,89 ; 130,60 ; 130,98 ; 138,08 (s, Cq) ; 67,39 ; 167,76 ; 170,92 (s, CO, CN et COO).

### Exemple 3b₈: point de fusion 81° C

RMN ¹H (CDCl₃) : 1,41 (s, 9H, CH₃) ; 3,72 (m, 3H, CH et CH₂Ph) ; 4,40 (d, ²J_{(HH)} = 16,7 Hz, 1H, CH₂COOtBu) ; 4,52 (d, ²J_{(HH)} = 16,7 Hz, 1H, CH₂COOtBu) ; 7,20-7,58 (m, 13H, Hₐᵣₒₘ).
RMN ¹³C (CDCl₃) : 27,79 (s, CH₃) ; 37,69 (s, CH₂Ph) ; 50,77 (s, N-CH₂COOtBu) ; 64,72 (s, CH) ; 82,33 (s, Cq(CH₃)₃) ; 122,69-131,45 (s, CHₐᵣₒₘ) ; 130,68 ; 131,10 ; 138,10 ; 138,91 ; 141,08 (s, Cq) ; 167,30 ; 167,47 ; 169,49 (s, CO, CN et COO).

### Exemple 3b₉: point de fusion 152° C

RMN ¹H (CDCl₃) : 1,41 (s, 9H, CH₃) ; 1,70 (d, ³J_{(HH)} = 6,4 Hz, 3H, CH-CH₃) ; 3,77 (q, ³J_{(HH)}= 6,4 Hz, 1H CH-CH₃) ; 4,23 (d, ²J_{(HH)} = 17,2 Hz, 1H, CH₂COOtBu) ; 4,51 (d, ²J_{(HH)} = 17,2 Hz, 1H, CH₂COOtBu) ; 7,21-7,60 (m, 8H, Hₐᵣₒₘ).
RMN ¹³C (CDCl₃) : 17,16 (s, CH₃) ; 27,81 (s, CH-CH₃) ; 50,65 (s, CH₂) ; 58,46 (s, CH) ; 82,25 (s, Cq(CH₃)₃) ; 122,29-131,37 (s, CHₐᵣₒₘ) ; 129,41 ; 130,82 ; 138,04 ; 141,18 (s, Cq) ; 167,04 ; 167,59 ; 170,50 (s, CO, CN et COO).

Les exemples 2b, 3a et 3b ci-dessus peuvent également être mis en oeuvre en utilisant un ylure supporté tel défini dans la présente l'invention, et plus particulièrement avec un polymère de formule (a) tel que définie ci-dessus dans laquelle m>1.

### Exemple 3c : C-alkylation de benzodiazépines en position 3 à l'aide d'un aldéhyde

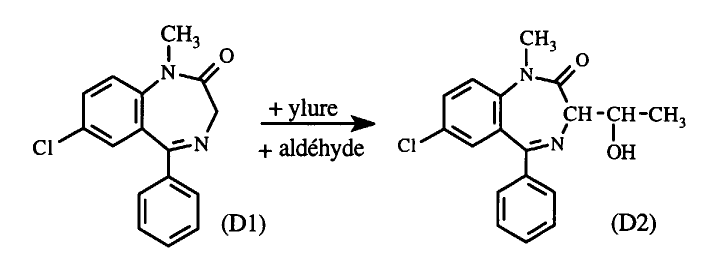

A une solution de 1 mmole du composé (D1) dans 5 ml de THF, on ajoute, à -78° C, 1,5 mmoles d'ylure (Me₂N)₃P=C(CH₃)₂ dans 7,5 ml de THF. Le mélange réactionnel est agité pendant 15 minutes puis, 1,5 mmoles d'éthanal sont additionnées. L'agitation est maintenue 30 minutes supplémentaires. Après extraction avec 60 ml d'éther, filtration et évaporation du solvant, l'huile est purifiée sur colonne chromatographique (éluant pentane/éther 95/5). Le composé de formule (D2) est obtenu sous forme d'une poudre (44 %).

## Revendications

1. Produits de formule générale I 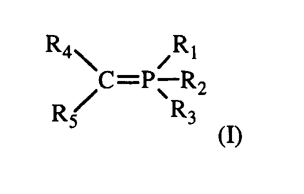 dans laquelle
R₁, R₂ et R₃ représentent, indépendamment, un radical alkyle inférieur ; alkoxy inférieur ; aryle éventuellement substitué par un ou plusieurs radicaux choisis parmi les radicaux suivants : alkyle inférieur, haloalkyle, alkoxy inférieur, halogène, cyano, nitro ou di(alkyl inférieur) amino ; ou amino de formule R'R''N dans laquelle R' et R'' représentent indépendamment l'un des radicaux non-substitués ou substitués (par un ou plusieurs substituants identiques ou différents) suivants : alkyle inférieur, alkoxy inférieur, cycloalkyle, aryl-alkyle inférieur, aryle, dans lesquels le substituant est un atome d'halogène ou un radical alkyle inférieur, alkoxy inférieur, cyano, nitro ou dialkylamino ;
R₄ représente l'atome d'hydrogène, un radical alkyle inférieur ; et
R₅ représente un support polymérique.

2. Produits selon la revendication 1 caractérisés en ce que R₁, R₂ et R₃ représentent, indépendamment, un radical alkoxy inférieur ou amino de formule RR'N telle que définie ci-dessus ; et R₄ représente l'atome d'hydrogène.

3. Produits selon l'une des revendications 1 à 2, caractérisés en ce que R₁, R₂ et R₃ représentent, indépendamment, le radical méthoxy, éthoxy, diméthylamino, (méthyl)(éthyl)amino ou diéthylamino.

4. Produits selon l'une des revendications 1 à 3, caractérisés en ce que le support polymérique R₅ est de type méthacrylique, acrylique tel que le polymère expansine®, ou polystyrénique.

5. Produits selon l'une des revendications 1 à 4, caractérisé en ce le support polymérique de type polystyrénique est de formule générale (s) 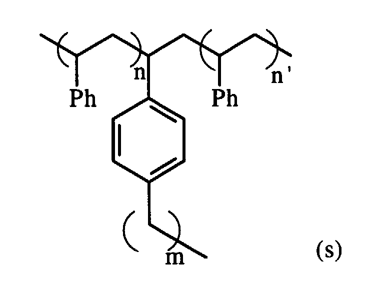 dans laquelle n, n', m sont des entiers supérieurs ou égaux à un.

6. Procédé de préparation des produits selon la revendication 1, caractérisé en ce que
l'on fait réagir une phosphine de formule générale (1)
PR₁R₂R₃ (1)
dans laquelle R₁, R₂ et R₃ ont la signification indiquée dans la revendication 1, avec un produit de formule générale (2) 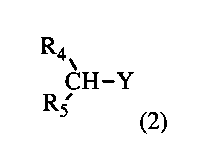 dans laquelle R₄ et R₅ ont la signification indiquée dans la revendication et Y est tel que Y⁻représente tout anion, pour obtenir le produit de formule générale (3) 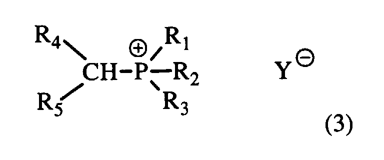 produit (3) ainsi obtenu que l'on traite avec une base forte pour obtenir un produit de formule I.

7. Utilisation de produits de formule générale I' 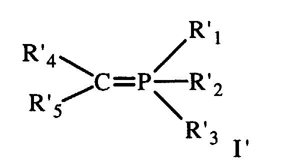 dans laquelle
R'₁, R'₂ et R'₃ représentent, indépendamment, un radical alkyle inférieur ; alkoxy inférieur ; aryle éventuellement substitué par un ou plusieurs radicaux choisis parmi les radicaux suivants : alkyle inférieur, haloalkyle, alkoxy inférieur, halogène, cyano, nitro ou di(alkyl inférieur) amino ; ou amino de formule R'R''N dans laquelle R' et R'' représentent indépendamment l'un des radicaux non-substitués ou substitués (par un ou plusieurs substituants identiques ou différents) suivants : alkyle inférieur, alkoxy inférieur, cycloalkyle, aryl-alkyle inférieur, aryle, dans lesquels le substituant est un atome d'halogène ou un radical alkyle inférieur, alkoxy inférieur, cyano, nitro ou dialkylamino ;
R'₄ représente l'atome d'hydrogène, un radical alkyle inférieur ou alkoxy inférieur ;
R'₅ représente l'atome d'hydrogène, un radical alkyle inférieur, alkoxy inférieur ou un support polymérique ;
en tant que base forte faiblement nucléophile.

8. Utilisation de produits de formule générale I' selon la revendication 7, caractérisée en ce que R'₁, R'₂ et R'₃ représentent, indépendamment, un radical alkoxy inférieur ou amino de formule RR'N telle que définie ci-dessus ; et R'₄ représente l'atome d'hydrogène.

9. Utilisation de produits de formule générale I' selon l'une des revendications 7 à 8, caractérisée en ce que R'₁, R'₂ et R'₃ représentent, indépendamment, le radical méthoxy, éthoxy, diméthylamino, (méthyle)(éthyle)amino, diéthylamino.

10. Utilisation de produits de formule générale I' selon l'une des revendications 7 à 9, caractérisée en ce que le support polymérique R'₅ est de type méthacrylique, acrylique tel que le polymère expansine®, ou polystyrénique.

11. Utilisation de produits de formule générale I' selon l'une des revendications 7 à 10, caractérisée en ce que le support polymérique est de formule générale (s) 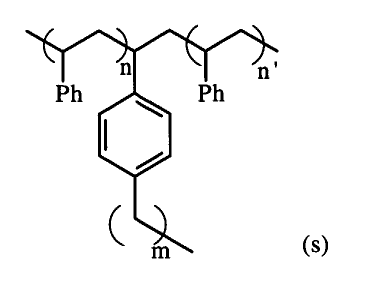 dans laquelle n, n', m sont des entiers supérieurs ou égaux à un.

12. Utilisation de produits de formule générale I' selon l'une des revendications 7 à 11, caractérisée en ce que les produits répondent aux formules suivantes :
- copolymère styrène/divinylbenzène-tris(diméthylamino)méthylènephosphorane;
- tris(diméthylamino)-C-diméthylméthylène phosphorane.

13. Utilisation selon l'une des revendications 7 à 12, dans les réactions de N-alkylation.

14. Utilisation selon l'une des revendications 7 à 13, dans les réactions de N-alkylation des lactames, des succinimides, d'oligopeptides et de benzodiazépines.

15. Utilisation selon l'une des revendications 7 à 12, dans les réactions de C-alkylation.

16. Utilisation selon l'une des revendications 7 à 12 et 15, dans les réactions de C-alkylation des lactames, des succinimides, de bases de Schiff et de benzodiazépines.

17. A titre de produits industriels nouveaux, les produits de formule (3) tel que définie à la revendication 5.
